(19) **Europäisches Patentamt**
**European Patent Office**
**Office européen des brevets**

(11) **EP 4 509 037 A1**

(12) **EUROPÄISCHE PATENTANMELDUNG**

(43) Veröffentlichungstag:
**19.02.2025 Patentblatt 2025/08**

(21) Anmeldenummer: **23192000.0**

(22) Anmeldetag: **17.08.2023**

(51) Internationale Patentklassifikation (IPC):
**A61B 5/00** $^{(2006.01)}$ **A61B 5/055** $^{(2006.01)}$
**A61B 6/04** $^{(2006.01)}$

(52) Gemeinsame Patentklassifikation (CPC):
**A61B 5/055; A61B 5/0077; A61B 5/704;**
**A61B 6/0492; A61B 6/08;** A61B 6/032;
A61B 6/0407

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Siemens Healthineers AG**
**91301 Forchheim (DE)**

(72) Erfinder:
• **Meise, Florian**
  **91353 Hausen (DE)**
• **Sukkau, Johann**
  **91074 Herzogenaurach (DE)**
• **Wohlers, Julian**
  **91052 Erlangen (DE)**

(74) Vertreter: **Siemens Healthineers**
**Patent Attorneys**
**Postfach 22 16 34**
**80506 München (DE)**

(54) **VERFAHREN UND VORRICHTUNG ZU EINEM ERFASSEN EINER POSITION EINES ZU UNTERSUCHENDEN BEREICHS FÜR EINE MEDIZINISCHE BILDGEBUNGSVORRICHTUNG**

(57) Die Erfindung geht aus von einem Verfahren zu einem Erfassen einer Position eines zu untersuchenden Bereichs eines Objekts für eine medizinische Bildgebungsuntersuchung, umfassend die folgenden Verfahrensschritte:
- Positionieren eines Markierelements an dem zu untersuchenden Bereich des Objekts, wobei das Positionieren des Markierelements manuell durch ein medizinisches Bedienpersonal erfolgt,
- Erfassen der Position des Markierelements mittels einer Erfassungsvorrichtung,
- Projizieren einer Feedback-Markierung an der erfassten Position des Markierelements mittels einer Projektionsvorrichtung, wobei bei der Ermittlung einer Projektionsposition der Feedback-Markierung ein Korrekturfaktor in z-Richtung berücksichtigt wird,
- Wiederholen des Erfassens der Position des Markierelements und des Projizierens der Feedback-Markierung, bis die Feedback-Markierung mit der Position des Markierelements übereinstimmt, und
- Festlegen der Position des Markierelements als Position des zu untersuchenden Bereiches des Objekts.

FIG 1

EP 4 509 037 A1

**Beschreibung**

Die vorliegende Erfindung betrifft ein Verfahren zu einem Erfassen einer Position eines zu untersuchenden Bereich eines Objekts für eine medizinische Bildgebungsuntersuchung. Des Weiteren betrifft die vorliegenden Erfindung auch eine medizinische Bildgebungsvorrichtung umfassend eine Erfassungsvorrichtung, die zu einem Erfassen einer Position eines Markierelements ausgebildet ist, einer Projektionsvorrichtung, die zu einer Projektion einer Feedback-Markierung an die erfasste Position des Markierelements ausgebildet ist, eine Kamera und eine Recheneinheit, wobei die medizinische Bildgebungsvorrichtung zu einer Ausführung eines Verfahrens zu einem Erfassen einer Position eines zu untersuchenden Bereichs eines Objekts für eine medizinische Bildgebungsuntersuchung ausgebildet ist.

Unabhängig vom grammatikalischen Geschlecht eines bestimmten Begriffes sind Personen mit männlicher, weiblicher oder anderer Geschlechteridentität mit umfasst.

Für eine medizinische Bildgebungsuntersuchung, insbesondere eine Magnetresonanzuntersuchung, ist es erforderlich, dass der Patient, insbesondere ein zu untersuchender Bereich des Patienten, korrekt im Isozentrum einer medizinischen Bildgebungsvorrichtung, beispielsweise einer Magnetresonanzvorrichtung, positioniert wird. Beispielsweise wird dabei der zu untersuchende Bereich des Patienten mit einem Laserstrahl, der an einer Frontseite einer Magnetresonanzvorrichtung angeordnet ist, markiert. Hierzu muss ein Liegentisch, auf dem der Patient für die Magnetresonanzuntersuchung positioniert ist, solange in z-Richtung, die der Längsrichtung des Liegentischs entspricht, verfahren werden, bis der zu untersuchende Bereich des Patienten mit der Lasermarkierung übereinstimmt.

Nach der Markierung des zu untersuchenden Bereichs wird anschließend automatisch der Liegentisch zusammen mit dem Patienten in einen Aufnahmebereich der Magnetresonanzvorrichtung gefahren, wobei hierbei der zu untersuchende Bereich bzw. der markierte Bereich des Patienten im Isozentrum positioniert wird. Jedoch ist eine derartige Positionierung für einen unerfahrenen und/oder ungeübten Benutzer sehr schwierig, so dass die Positionierung einen hohen Zeitaufwand benötigt und/oder es auch zu Fehlpositionierungen kommen kann.

Aus DE 10 2021 202 978 A1 ist eine Vorrichtung zur Erfassung und Markierung eines zu untersuchenden Bereichs eines Patienten bekannt, wobei die Vorrichtung an einer Decke über dem Liegentisch angeordnet ist. Hierbei wird der zu untersuchende Bereich von einem Benutzer mit einem Markierobjekt markiert und von einer Erfassungsvorrichtung erfasst. Anschließend erfolgt eine Projektion einer Feedback-Markierung an der erfassten Position mittels einer Projektionsvorrichtung. Wenn die Position der Feedback-Markierung mit der Position des Markierobjekts übereinstimmt, wird anhand dieser Position eine Verschiebung des Liegentischs bis zum Isozentrum ermittelt.

Ist jedoch die Projektionsvorrichtung fest an der Decke des Untersuchungsraums angeordnet, weist ein derartiges Verfahren den Nachteil auf, dass in z-Richtung Projektionsverzerrungen auftreten, die umso größer sind, je höher ein Profil des Patienten am Projektionsort und je weiter entfernt der zu untersuchende bezogen auf die Position der Projektionsvorrichtung ist.

Der vorliegenden Erfindung liegt insbesondere die Aufgabe zugrunde, schnelle und exakte Markierung des zu untersuchenden Bereichs bereitzustellen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Erfassen einer Position eines zu untersuchenden Bereichs eines Objekts für eine medizinische Bildgebungsuntersuchung, umfassend die folgenden Verfahrensschritte:

- Positionieren eines Markierelements an dem zu untersuchenden Bereich des Objekts, wobei das Positionieren des Markierelements manuell durch ein medizinisches Bedienpersonal erfolgt,
- Erfassen der Position des Markierelements mittels einer Erfassungsvorrichtung,
- Projizieren einer Feedback-Markierung an der erfassten Position des Markierelements mittels einer Projektionsvorrichtung, wobei bei der Ermittlung einer Position der Feedback-Markierung ein Korrekturfaktor in z-Richtung berücksichtigt wird,
- Wiederholen des Erfassens der Position des Markierelements und des Projizierens der Feedback-Markierung, bis die Feedback-Markierung mit dem zu untersuchenden Bereichs des Objekts übereinstimmt, und
- Festlegen der Position des Markierelements als Position des zu untersuchenden Bereichs.

Die medizinische Bildgebungsuntersuchung kann dabei eine Computertomografie-Untersuchung (CT-Untersuchung), eine PET-Untersuchung (Positron-Emissions-Tomografie-Untersuchung), eine Magnetresonanzuntersuchung oder auch eine Kombination von mehreren medizinischen Bildgebungsuntersuchungen umfassen, wie beispielsweise eine Magnetresonanz-PET-Untersuchung oder eine CT-PET-Untersuchung. Dementsprechend kann auch die medizinische Bildgebungsvorrichtung, mit der ein derartiges Verfahren durchgeführt wird, eine Computertomografie-Vorrichtung (CT-Vorrichtung), eine PET-Vorrichtung (Positron-Emissions-Tomografie-Vorrichtung), eine Magnetresonanzvorrichtung oder eine Kombination von mehreren medizinischen Bildgebungsvorrichtung umfassen, wie beispielsweise eine Magnetresonanz-PET-Vorrichtung oder eine CT-PET-Vorrichtung umfassen.

Das zu untersuchende Objekt umfasst bevorzugt einen Patienten. Insbesondere soll mittels der medizinischen Bildgebungsuntersuchung eine klinische und/diagnostische Fragestellung am Patienten geklärt werden. Zudem kann das Objekt auch ein Phantom für beispielsweise Justage-Messungen und/oder weitere, dem Fachmann als sinnvoll erscheinende Objekte umfassen.

Der zu untersuchende Bereich des Objekts, insbesondere des Patienten, umfasst dabei denjenigen Bereich, für den eine klinische und/diagnostische Fragestellung geklärt werden soll. Beispielsweise kann der zu untersuchende Bereich ein Organ, ein Gelenk, einen Kopf usw. eines Patienten umfassen.

Zur Erfassung des zu untersuchenden Bereichs wird vom Benutzer bevorzugt ein Markierelement auf dem zu untersuchenden Bereich des Objekts und/oder des Patienten positioniert, wobei das Markierelement von der Erfassungsvorrichtung als dieses erkannt und dessen Position mittels der Erfassungsvorrichtung erfasst wird. Das Markierelement kann beispielsweise einen Finger des medizinischen Bedienpersonals oder auch einen handgeführten Gegenstand, beispielsweise einen Markierstab, umfassen. Bevorzugt wird dabei eine Spitze des Fingers und/oder eine Spitze des handgeführten Markierelements als die Position des Markierelements festgelegt. Alternativ oder zusätzlich kann der zu untersuchende Bereich auch auf einem Display, beispielsweise an einem Touch-Display, von dem medizinischen Bedienpersonal festgelegt und/oder markiert werden.

Die Erfassungsvorrichtung ist dazu ausgebildet, eine Position des zu untersuchenden Bereichs des Objekts, insbesondere des Patienten, für eine anstehende medizinische Bildgebungsuntersuchung zu erfassen. Dabei kann die Erfassungsvorrichtung eine Kamera, beispielsweise eine 2D-Kamera, bevorzugt zumindest zwei 2D-Kameras, oder eine 3D-Kamera umfassen. Zudem kann die Erfassungsvorrichtung auch mehr als eine Kamera umfassen, wie beispielsweise ein Kamera-Array. Die Erfassungsvorrichtung ist bevorzugt an einer Decke eines Untersuchungsraums, in dem die medizinische Bildgebungsvorrichtung angeordnet ist, angeordnet. Insbesondere ist die Erfassungsvorrichtung in einem Frontbereich vor einer Frontseite der Scannereinheit angeordnet. Der Frontbereich befindet sich dabei an die Frontseite angrenzend vor der Scannereinheit. Zudem kann die Erfassungsvorrichtung weitere Einheiten und/oder Sensoren zur Erfassung des zu untersuchenden Bereichs umfassen, die beispielswiese an einem Liegentisch der medizinischen Bildgebungsvorrichtung angeordnet sein können.

Die Projektionsvorrichtung umfasst bevorzugt einen Videoprojektor und/oder eine Laser-Markierungseinheit, die zu einer Projektion und/oder Anzeige einer Feedback-Markierung an der von der Erfassungsvorrichtung erfassten Position des Markierelements ausgebildet ist. Die Feedback-Markierung umfasst bevorzugt eine Markierungslinie, deren z-Position in z-Richtung durch das Markierelement festgelegt wird, wobei die Markierungslinie in x-Richtung verläuft. Bevorzugt verläuft dabei die Markierungslinie in x-Richtung über die gesamte Breite des Liegentischs. Die z-Richtung entspricht dabei der Längsrichtung des Liegentisch und/oder einer Einfahrrichtung des Liegentischs in den Aufnahmebereich. Die x-Richtung ist dabei quer zur z-Richtung in horizontaler Richtung ausgerichtet. Mittels der Feedback-Markierung erhält der Benutzer ein Feedback, ob der zu untersuchende Bereich des Objekts und/oder Patienten korrekt erfasst wurde. Die Projektionsvorrichtung ist bevorzugt an einer Decke des Untersuchungsraums angeordnet. Insbesondere ist die Projektionsvorrichtung in einem Frontbereich vor einer Frontseite der Scannereinheit angeordnet.

Da die Projektionsvorrichtung fest an der Decke des Untersuchungsraums angeordnet ist, tritt bei der Projektion der Feedback-Markierung eine Verzerrung auf, die umso stärker ausgeprägt ist, je weiter entfernt das Markierelement und damit der zu untersuchende Bereich von einer Position der Projektionsvorrichtung bezogen auf die z-Richtung ist. Zudem ist die Verzerrung auch von einer Höhe eines Profils des Objekts an der Position des Markierelements und damit des zu untersuchende Bereichs abhängig. Der Korrekturfaktor korrigiert dabei die Verzerrung in z-Richtung, so dass die Feedback-Markierung an der Position des Markierelements und damit am zu untersuchenden Bereich erscheint.

Die Verfahrensschritte des Erfassens der Position des Markierelements und des Projizierens der Feedback-Markierung werden so lange wiederholt, bis die Feedback-Markierung mit der Position des Markierelements und damit der Position des zu untersuchenden Bereichs des Objekts übereinstimmt. Beispielsweise kann nach Erhalt der Feedback-Markierung, sofern diese nicht mit einer Position des zu untersuchenden Bereichs des Objekts, insbesondere des Patienten, übereinstimmt, durch eine manuelle Neupositionierung des Markierelements der Erfassungsvorrichtung mitgeteilt werden, dass die erfasste Position nicht korrekt war. Dagegen kann ein Beibehalten der Position des Markierelements und/oder ein komplettes Wegnehmen des Markierelements aus dem Erfassungsbereich der Erfassungsvorrichtung der Erfassungsvorrichtung signalisieren, dass die Erfassung der Position des Markierelements abgeschlossen ist. Sobald die Position der Feedback-Markierung mit der Position des Markierelements und damit des zu untersuchenden Bereichs des Patienten für den Benutzer übereinstimmt und er der Erfassungsvorrichtung signalisiert, dass die Position korrekt ist, wird die Position des Markierelements als die Position des zu untersuchenden Bereichs des Objekts festgelegt.

Die Erfassungsvorrichtung weist hierzu eine Recheneinheit und/oder eine Steuereinheit mit einer entsprechenden Software auf, die aus den mittels der Erfassungsvorrichtung erfassten Bilddaten das Markierelement erkennt. Die Recheneinheit und/oder Steuereinheit der Erfassungsvorrichtung ist dazu ausgebildet eine Position und/oder Positionsänderung des Markierelements zu erkennen und zu bestimmten. Weiterhin ist die Recheneinheit und/oder die Steuereinheit der Erfassungsvorrichtung dazu ausgebildet, ein Beenden des Erfassungsvorgangs zu erkennen, beispielsweise durch ein Beibehalten der Position des Markierelements und/oder ein komplettes Entfernen des Markierelement aus dem Erfassungsbereich der Erfassungsvorrichtung.

Das erfindungsgemäße Verfahren wird von einer Recheneinheit einer medizinischen Bildgebungsvorrichtung gesteuert. Die Recheneinheit umfasst zumindest ein Rechenmodul und/oder einen Prozessor, wobei die Recheneinheit einem Steuern des Verfahrens ausgebildet ist. So ist insbesondere die Recheneinheit dazu ausgebildet, computerlesbare Instruktionen auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen. Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Insbesondere ist die Recheneinheit dazu ausgebildet, die Projektionsvorrichtung entsprechend zu steuern, dass diese ihren Projektionsbereich auf den mittels der Erfassungsvorrichtung erfassten Position des Markierelements richtet. Insbesondere ist die Recheneinheit auch dazu ausgebildet, den Korrekturfaktor für die Projektion der Feedback-Markierung zu bestimmen und bei der Übermittlung der Positionsinformation der Feedback-Markierung an die Projektionsvorrichtung bereits zu berücksichtigen. Dabei kann die Projektionsvorrichtung bereits eine korrigierte Position für die Feedback-Markierung erhalten. Zudem kann die Recheneinheit auch eine Kommunikation zwischen der Erfassungsvorrichtung und der Projektionsvorrichtung steuern.

Die Erfindung weist den Vorteil auf, dass eine schnelle und exakte Markierung des zu untersuchenden Bereichs für eine medizinische Bildgebungsuntersuchung bereitgestellt werden kann. Des Weiteren kann durch die korrekte und/oder exakte Markierung des Markierelements und damit des zu untersuchenden Bereichs des Objekts eine korrekte Positionierung des zu untersuchenden Bereichs innerhalb eines Patientenaufnahmebereichs einer medizinischen Bildgebungsvorrichtung erreicht werden und damit auch unerwünschte Fehlpositionierungen verhindert werden. Damit können auch medizinische Bildgebungsuntersuchungen einfach und schnell durchgeführt werden und Wiederholungen von einzelnen Messungen aufgrund einer Fehlpositionierung reduziert und/oder vermieden werden. Insbesondere kann derart ein medizinisches Bedienpersonal vorteilhaft in einem Workflow zur Positionierung des zu untersuchenden Bereichs innerhalb des Isozentrums vorteilhaft unterstützt werden.

Ein weiterer Vorteil ist, dass zur Eingabe und/oder Erfassung des zu untersuchenden Bereichs des Objekts keine Benutzereingabe an einer Eingabeeinheit getätigt werden muss, beispielsweise ein Berühren eines Touch-Displays, so dass besonders einfach Hygienestandards während einer medizinischen Bildgebungsuntersuchung eingehalten werden können.

In einer vorteilhaften Weiterbildung des erfindungsgemäßen Verfahrens kann es vorgesehen sein, dass zur Ermittlung des Korrekturfaktors 3D-Daten des Objekts erfasst werden und anhand der 3D-Daten ein 3D-Profil des Objekts bestimmt wird. Bevorzugt erfolgt die Erfassung der 3D-Daten des Objekts mittels einer Kamera. Bevorzugt ist die Kamera dabei von der Erfassungsvorrichtung umfasst. In einer alternativen Ausgestaltung kann die Kamera auch getrennt zur Erfassungsvorrichtung ausgebildet sein. Die Kamera kann dabei eine 3D-Kamera zur Erfassung des 3D-Profils umfassen. Alternativ hierzu können auch zumindest zwei 2D-Kameras für die Erfassung des 3D-Profils verwendet werden. Für die Bestimmung eines 3D-Profils des Objekts, insbesondere eines Patienten, aus den 3D-Daten weist die Recheneinheit der medizinischen Bildgebungsvorrichtung eine entsprechende Auswertesoftware auf. Aus dem 3D-Profil kann vorteilhaft eine Höhe des Objekts, insbesondere des Patienten, bestimmt werden, die dieser auf einer Liegefläche des Patiententischs liegend bezogen auf die Liegefläche aufweist, und für die Ermittlung des Korrekturfaktors bereitgestellt werden.

In einer vorteilhaften Weiterbildung des erfindungsgemäßen Verfahrens kann es vorgesehen sein, dass aus dem 3D-Profil eine Höhe des Objekts an der erfassten Position des Markierelements ermittelt wird. Die Höhe des Objekts, insbesondere des Patienten, an der erfassten Position des Markierelements umfasst dabei eine Erstreckung des Objekts, insbesondere des Patienten, in y-Richtung und/oder vertikaler Richtung bezogen auf eine Liegefläche des Liegentischs, auf dem das Objekt, insbesondere der Patient, für eine medizinische Bildgebungsuntersuchung angeordnet ist. Bevorzugt erfolgt die Ermittlung der Höhe des Objekts, insbesondere des Patienten, bezogen auf die Liegefläche des Liegentischs mittels der Recheneinheit der medizinischen Bildgebungsvorrichtung. Die Ermittlung der Höhe des Objekts, insbesondere des Patienten, bezogen auf die Liegefläche des Liegentischs an der erfassten Position des Markierelements ermöglicht eine besonders exakte Ermittlung eines Korrekturfaktors, der bei der Projektion der Feedback-Markierung berücksichtigt wird.

In einer vorteilhaften Weiterbildung des erfindungsgemäßen Verfahrens kann es vorgesehen sein, dass die Feedback-Markierung zur Festlegung der Position des zu untersuchenden Bereichs in z-Richtung eine projizierte Markierungslinie in x-Richtung auf dem Objekt umfasst, wobei für jede Position in x-Richtung der Markierungslinie eine Höhe des Objekts aus dem 3D-Profil des Objekts ermittelt wird. Derart kann für jede Position x-Richtung ein Korrekturfaktor ermittelt und/oder bereitgestellt werden. Dabei können die einzelnen Korrekturfaktoren für unterschiedliche Positionen in x-Richtung unterschiedlich sein. Dies ermöglicht auch, dass die Markierungslinie unabhängig von dem Profil des Objekts, insbesondere des Patienten, eine gerade Linie in x-Richtung für das medizinische Bedienpersonal markiert.

In einer vorteilhaften Weiterbildung des erfindungsgemäßen Verfahrens kann es vorgesehen sein, dass der Korrekturfaktor in z-Richtung eine Höhe des Objekts an der erfassten Position des Markierelements umfasst. Bevorzugt umfasst Korrekturfaktor eine Höhe des Objekts, insbesondere des Patienten, für jede Position in x-Richtung. Dies ermöglicht, dass die Markierungslinie unabhängig von dem Profil des Objekts, insbesondere des Patienten, eine gerade Linie in x-Richtung für das medizinische Bedienpersonal markiert.

In einer vorteilhaften Weiterbildung des erfindungsgemäßen Verfahrens kann es vorgesehen sein, dass der Korrekturfaktor einen Abstand des Markierelements in z-Richtung bezüglich der Position der Projektionsvorrichtung und eine Höhe der Projektionsvorrichtung über dem Liegentisch umfasst. Der Korrekturfaktor für jede Position in x-Richtung berechnet sich dabei wie folgt:

$$KF_x = \frac{d}{c} H_x \, .$$

Hierbei ist $KF_x$ der Korrekturfaktor und/oder ein Korrekturwert an der Position x, d ist der Abstand der erfassten Position des Markierelements in z-Richtung. C umfasst die Höhe der Projektionsvorrichtung über dem Liegentisch. $H_x$ ist die Höhe des Objekts, insbesondere des Patienten, in an der Position x bezogen auf die Liegefläche des Liegentischs. Der Korrekturfaktor für jede Position in x-Richtung an der Position des Markierelements wird bevorzugt mittels der Recheneinheit der medizinischen Bildgebungsvorrichtung ermittelt. Derart kann der Korrekturfaktor für jede Position in x-Richtung besonders exakt und profilabhängig und/oder höhenabhängig ermittelt werden. Insbesondere kann derart eine Fehlinterpretation der Feedback-Markierung verhindert werden und damit auch eine Fehlpositionierung für die medizinische Bildgebungsuntersuchung.

In einer vorteilhaften Weiterbildung des erfindungsgemäßen Verfahrens kann es vorgesehen sein, dass anhand von 3D-Daten eines Patienten ein Augenbereich des Patienten ermittelt wird, wobei der Augenbereich des Patienten bei der Projektion der Feedback-Markierung von einem Projektionsbereich der Projektionsvorrichtung ausgeschlossen wird. Der Projektionsbereich der Projektionsvorrichtung umfasst bevorzugt einen Bereich auf dem Objekt, an dem die Feedback-Markierung projiziert wird. Insbesondere wird der Augenbereich des Patienten aus den 3D-Daten mittels der Recheneinheit der medizinischen Bildgebungsvorrichtung ermittelt, die hierzu eine entsprechende Software aufweist. Derart kann eine hohe Sicherheit bei der Projektion der Feedback-Markierung für einen Patienten erreicht werden. Insbesondere kann derart ein Blenden eines Patienten und/oder bei einer Ausbildung der Projektionsvorrichtung mit einer Lasermarkierungsvorrichtung ein Verbrennen der Netzhaut von Patienten vorteilhaft verhindert werden.

In einer vorteilhaften Weiterbildung des erfindungsgemäßen Verfahrens kann es vorgesehen sein, dass anhand der erfassten Position des zu untersuchenden Bereichs des Objekts eine Untersuchungsposition des Liegentischs berechnet wird. Dabei erfolgt das Berechnen der Untersuchungsposition des Liegentischs mittels der Recheneinheit der medizinischen Bildgebungsvorrichtung. Die Untersuchungsposition umfasst dabei diejenige Position des Liegentischs bezüglich der Scannereinheit, dass der zu untersuchende Bereich des Objekts, insbesondere des Patienten, innerhalb des Isozentrums der medizinischen Bildgebungsvorrichtung für die anstehende medizinische Bildgebungsuntersuchung angeordnet ist. Derart kann eine einfache und zeitsparende Positionierung des zu untersuchenden Bereichs im Isozentrum der medizinischen Bildgebungsvorrichtung ermöglicht werden. Insbesondere kann derart das medizinische Bedienpersonal vorteilhaft bei der Positionierung eines Patienten innerhalb der medizinischen Bildgebungsvorrichtung unterstützt werden und ein Positionierungsworkflow für unerfahrene Benutzer vereinfacht werden.

In einer vorteilhaften Weiterbildung des erfindungsgemäßen Verfahrens kann es vorgesehen sein, dass der Liegentisch automatisch in die Untersuchungsposition gefahren wird für eine Erfassung von medizinischen Bildgebungsdaten des zu untersuchenden Bereichs des Objekts. Hierbei kann ebenfalls eine einfache und zeitsparende Positionierung des zu untersuchenden Bereichs im Isozentrum der medizinischen Bildgebungsvorrichtung ermöglicht werden. Vorzugsweise weist hierzu die Patientenlagerungsvorrichtung eine entsprechende Horizontaleinstelleinheit und eine Positionssteuereinheit auf zu Positionierung des Liegentischs in der Untersuchungsposition.

Des Weiteren geht die Erfindung aus von einer medizinischen Bildgebungsvorrichtung umfassend eine Erfassungsvorrichtung, die zu einem Erfassen einer Position eines Markierelements ausgebildet ist, eine Projektionsvorrichtung, die zu einer Projektion einer Feedback-Markierung an der erfassten Position des Markierelements ausgebildet ist, eine Kamera und eine Recheneinheit, wobei die medizinische Bildgebungsvorrichtung zu einer Ausführung eines Verfahrens zu einem Erfassen einer Position eines zu untersuchenden Bereichs eines Objekts für eine medizinische Bildgebungsuntersuchung ausgebildet ist.

Die medizinische Bildgebungsvorrichtung ist bevorzugt zu einem Erfassen von medizinischen und/oder diagnostischen Bilddaten eines Patienten und/oder Objekts ausgelegt und/oder ausgebildet.

Die medizinische Bildgebungsvorrichtung umfasst bevorzugt eine Scannereinheit zur Erfassung der medizinischen und/oder diagnostischen Bilddaten. Die Scannereinheit umgibt dabei einen Aufnahmebereich der medizinischen Bildgebungsvorrichtung. Der Aufnahmebereich ist bevorzugt zylinderförmig ausgebildet und zu einer Aufnahme des Patienten und/oder des Objekts, insbesondere des zu untersuchenden Bereichs des Patienten und/oder Objekts, für eine medizinische Bildgebungsuntersuchung ausgebildet.

Für eine medizinische Bildgebungsuntersuchung wird das Objekt, insbesondere der Patient, insbesondere der zu untersuchende Bereich des Objekts, insbesondere des Patienten, innerhalb des Aufnahmebereichs der medizinischen Bildgebungsvorrichtung positioniert. Innerhalb des Aufnahmebereichs ist bevorzugt das Field of View (FOV) und/oder ein Isozentrum der medizinischen Bildgebungsvorrichtung angeordnet. Das FOV umfasst bevorzugt einen Erfassungsbereich der medizinischen Bildgebungsvorrichtung, innerhalb dessen die Bedingungen für eine Erfassung von medizinischen Bilddaten innerhalb des Aufnahmebereichs vorliegen, wie beispielsweise ein homogenes Grundmagnetfeld bei einer Ausbildung der medizinischen Bildgebungsvorrichtung als Magnetresonanzvorrichtung. Das Isozentrum der medizinischen Bildgebungsvorrichtung umfasst bevorzugt den Bereich und/oder Punkt innerhalb der medizinischen Bildgebungsvorrichtung, der die optimalen und/oder idealen Bedingungen für die Erfassung von medizinischen Bilddaten aufweist. Beispielsweise umfasst bei einer Ausbildung der medizinischen Bildgebungsvorrichtung als Magnetresonanzvorrichtung das Isozentrum den homogensten Magnetfeldbereich innerhalb der Magnetresonanzvorrichtung.

Zu einem Einfahren und/oder Positionieren des Objekts und/oder des Patienten, insbesondere des zu untersuchenden Bereichs des Objekts und/oder des Patienten, innerhalb des Aufnahmebereichs umfasst die medizinische Bildgebungsvorrichtung eine Patientenlagerungsvorrichtung. Die Patientenlagerungsvorrichtung weist dabei einen Liegentisch auf, wobei der Liegentisch relativ zur Scannereinheit bewegbar ausgebildet ist. Bevorzugt ist hierbei der Liegentisch in Längsrichtung des Liegentischs und/oder in Längsrichtung des Aufnahmebereichs innerhalb des Aufnahmebereichs bewegbar ausgebildet, um den Patienten in einer Untersuchungsposition innerhalb des Aufnahmebereichs für eine medizinische Bildgebungsuntersuchung zu positionieren.

Die medizinische Bildgebungsvorrichtung kann dabei zumindest eine als 3D-Kamera ausgebildete Kamera umfassen. Alternativ hierzu kann die medizinische Bildgebungsvorrichtung auch zumindest zwei als 2D-Kamera ausgebildete Kameras umfassen.

Die erfindungsgemäße medizinische Bildgebungsvorrichtung weist den Vorteil auf, dass eine schnelle und exakte Markierung des zu untersuchenden Bereichs für eine medizinische Bildgebungsuntersuchung bereitgestellt werden kann. Des Weiteren kann durch die korrekte und/oder exakte Markierung des Markierelements und damit des zu untersuchenden Bereichs des Objekts eine korrekte Positionierung des zu untersuchenden Bereichs innerhalb eines Patientenaufnahmebereichs einer medizinischen Bildgebungsvorrichtung erreicht werden und damit auch Fehlpositionierungen verhindert werden. Damit können auch medizinische Bildgebungsuntersuchungen einfach und schnell durchgeführt werden und Wiederholungen von einzelnen Messungen aufgrund einer Fehlpositionierung verhindert werden. Insbesondere kann derart ein medizinisches Bedienpersonal vorteilhaft in einem Workflow zur Positionierung des zu untersuchenden Bereichs innerhalb des Isozentrums vorteilhaft unterstützt werden.

Ein weiterer Vorteil ist, dass zur Eingabe und/oder Erfassung des zu untersuchenden Bereichs des Objekts keine Benutzereingabe an einer Eingabeeinheit getätigt werden muss, beispielsweise ein Berühren eines Touch-Displays, so dass besonders einfach Hygienestandards während einer medizinischen Bildgebungsuntersuchung eingehalten werden können.

Die Vorteile der erfindungsgemäßen medizinischen Bildgebungsvorrichtung entsprechen im Wesentlichen den Vorteilen des erfindungsgemäßen Verfahrens zu einem Erfassen einer Position eines zu untersuchenden Bereichs eines Objekts für eine medizinische Bildgebungsuntersuchung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung der erfindungsgemäßen medizinischen Bildgebungsvorrichtung kann es vorgesehen sein, dass die medizinische Bildgebungsvorrichtung eine Scannereinheit mit einer Frontseite umfasst, wobei die Erfassungsvorrichtung und/oder die Projektionsvorrichtung in einem Frontbereich vor der Frontseite der Scannereinheit angeordnet ist oder sind. In diesem Frontbereich vor der Frontseite der Scannereinheit ist der Liegentisch der Patientenlagerungsvorrichtung zusammen mit dem zu untersuchenden Objekt, insbesondere mit dem zu untersuchenden Patienten, während einer Vorbereitung des Objekts, insbesondere des Patienten, für die anstehende medizinische Bildgebungsuntersuchung angeordnet. Diese Ausgestaltung ermöglicht eine vorteilhafte Anordnung der Erfassungsvorrichtung und/oder der Projektionsvorrichtung im Hinblick auf eine Erfassung des zu untersuchenden Bereichs für die medizinische Bildgebungsuntersuchung.

In einer vorteilhaften Weiterbildung der erfindungsgemäßen medizinischen Bildgebungsvorrichtung kann es vorgesehen sein, dass die Erfassungsvorrichtung und/oder die Projektionsvorrichtung fest an einer Decke eines Untersuchungsraums und/oder an der Frontseite der Scannereinheit angeordnet ist oder sind. Innerhalb des Untersuchungsraums ist die Scannereinheit der medizinischen Bildgebungsvorrichtung angeordnet. Eine Steuereinheit zur Steuerung der Scannereinheit kann dabei ebenfalls innerhalb des Untersuchungsraums angeordnet sein. Bei einer Ausbildung der medizinischen Bildgebungsvorrichtung als Magnetresonanzvorrichtung ist die Steuereinheit zur Steuerung der Magnetresonanzvorrichtung innerhalb eines Kontrollraums angeordnet, der getrennt zum Untersuchungsraum ausgebildet ist. Insbesondere ist der Kontrollraum vom Untersuchungsraum hinsichtlich einer Hochfrequenzstrahlung abgeschirmt.

Ist die Erfassungsvorrichtung und/oder die Projektionsvorrichtung an der Frontseite der Scannereinheit der medizinischen Bildgebungsvorrichtung angeordnet, dann ist die Erfassungsvorrichtung und/oder die Projektionsvorrichtung bevorzugt über eine Einführöffnung des Patientenaufnahmebereichs der Scannereinheit angeordnet. Dies ermöglicht eine vorteilhafte Ausrichtung eines Sichtfelds und/oder Erfassungsfelds der Erfassungsvorrichtung und/oder der Projektionsvorrichtung auf den Liegentisch, insbesondere eine Liegefläche des Liegentischs, während einer Vorbereitung des Patienten.

Die Erfassungsvorrichtung kann zwar ein Erfassungsfeld und/oder Sichtfeld der Erfassungsvorrichtung in unterschiedliche Richtungen ausrichten, beispielsweise durch ein Drehen einer Kamera der Erfassungsvorrichtung, jedoch ist Erfassungsvorrichtung an einem fixen Punkt an der Decke des Untersuchungsraums angeordnet und/oder befestigt. Ebenso kann auch die Projektionsvorrichtung zwar ein Sichtfeld und/oder Projektionsfeld der Projektionsvorrichtung in unterschiedliche Richtungen ausrichten, beispielsweise durch ein Drehen einer Lasereinheit der Projektionsvorrichtung, jedoch ist die Anordnung und/oder Befestigung der Projektionsvorrichtung an einem fixen Punkt an der Decke des Untersuchungsraums.

Diese Ausgestaltung der Erfindung ermöglicht eine platzsparende Anordnung der Erfassungsvorrichtung und/oder der Projektionsvorrichtung an der Decke des Untersuchungsraums. Insbesondere können derart auch zusätzlich Kosten, die eine mobile und/oder verfahrbare Anordnung der Erfassungsvorrichtung und/oder der Projektionsvorrichtung an der Decke des Untersuchungsraums mit sich brächte, eingespart werden.

In einer vorteilhaften Weiterbildung der erfindungsgemäßen medizinischen Bildgebungsvorrichtung kann es vorgesehen sein, dass die Erfassungsvorrichtung die Kamera umfasst. Derart kann eine besonders kompakte und bauteilesparende Erfassung der Position des zu untersuchenden Bereichs bereitgestellt werden. In einer alternativen Ausgestaltung kann die Kamera auch separat zur Erfassungsvorrichtung ausgebildet sein. Die Kamera kann dabei zumindest eine 3D-Kamera oder auch mehrere 2D-Kameras umfassen. Insbesondere kann mittels zumindest zwei 2D-Kameras und damit aus zumindest zwei unterschiedlichen 2D-Bildern des zu untersuchenden Bereichs unter Ausnutzung von Stereoskopie-Effekten ein 3D-Profil des Patienten, insbesondere des zu untersuchenden Bereichs des Patienten, ermittelt werden.

In einer vorteilhaften Weiterbildung der erfindungsgemäßen medizinischen Bildgebungsvorrichtung kann es vorgesehen sein, die medizinische Bildgebungsvorrichtung als Magnetresonanzvorrichtung ausgebildet ist. Insbesondere bei Magnetresonanzuntersuchungen, die eine längere Zeit beanspruchen, ist eine exakte Positionierung besonders wichtig. Damit wird auch die Aufenthaltszeit eines Patienten innerhalb des Aufnahmebereichs auf die Messzeit reduziert und Messwiederholungen aufgrund von Fehlpositionierungen können somit verhindert werden.

Die Magnetresonanzvorrichtung umfasst bevorzugt eine als Magneteinheit ausgebildete Scannereinheit zur Erfassung der medizinischen und/oder diagnostischen Bilddaten. Hierbei umfasst die Magneteinheit einen Grundmagneten, eine Gradientenspuleneinheit und eine Hochfrequenzantenneneinheit. Die Hochfrequenzantenneneinheit ist hierbei fest innerhalb der Magneteinheit angeordnet. Die Magneteinheit umgibt dabei einen Aufnahmebereich der Magnetresonanzvorrichtung. Der Aufnahmebereich ist bevorzugt zylinderförmig ausgebildet und zu einer Aufnahme des Patienten und/oder des Objekts, insbesondere des zu untersuchenden Bereichs des Patienten und/oder Objekts, für eine Magnetresonanzuntersuchung ausgebildet.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Die Gradientenspuleneinheit ist zu einer Erzeugung von Magnetfeldgradienten, die für eine Ortskodierung während einer Bildgebung verwendet werden, ausgebildet. Die Hochfrequenzantenneneinheit ist zu einer Aussendung von Hochfrequenzpulsen und/oder Anregungspulsen zur Generierung von Magnetresonanzsignalen ausgebildet.

Des Weiteren geht die Erfindung aus von einem Computerprogrammprodukt, welches ein Programm umfasst und direkt in einem Speicher einer programmierbaren Recheneinheit ladbar ist, mit Programmmitteln, um ein Verfahren zu einem Erfassen einer Position eines zu untersuchenden Bereichs eines Objekts für eine medizinische Bildgebungsuntersuchung auszuführen, wenn das Programm in der Recheneinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:

Fig. 1 eine erfindungsgemäße medizinische Bildgebungsvorrichtung mit einer Erfassungsvorrichtung und einer Projektionsvorrichtung,

Fig. 2 ein erfindungsgemäßes Verfahren zu einem Erfassen einer Position eines zu untersuchenden Bereichs eines Objekts für eine medizinische Bildgebungsuntersuchung,

Fig. 3 eine Detailansicht der Projektionsvorrichtung mit dem Liegentisch und einem auf dem Liegentisch positionierten Objekt, und

Fig. 4 eine Projektion einer Markierungslinie auf dem Objekt ohne Berücksichtigung eines Korrekturfaktors und eine Projektion einer Markierungslinie unter Berücksichtigung des Korrekturfaktors.

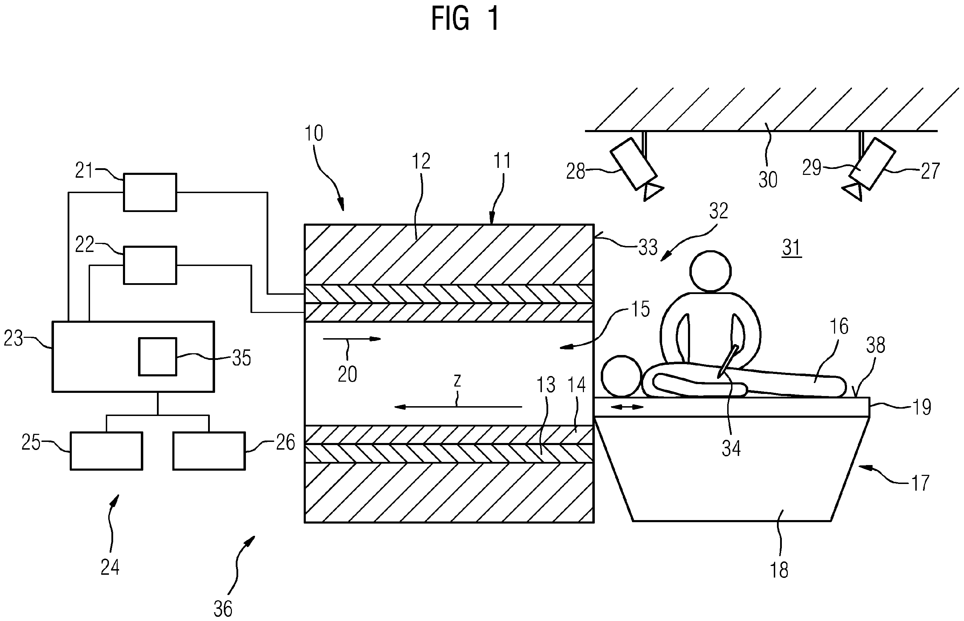

In Fig. 1 ist eine medizinische Bildgebungsvorrichtung 36 schematisch dargestellt. Die medizinische Bildgebungsvorrichtung 36 ist im vorliegenden Ausführungsbeispiel von einer Magnetresonanzvorrichtung 10 gebildet, wobei beispielhaft die vorliegende Erfindung anhand der Magnetresonanzvorrichtung 10 erläutert wird. Die vorliegende Erfindung ist jedoch nicht auf Ausgestaltung der medizinischen Bildgebungsvorrichtung 36 auf eine Magnetresonanzvorrichtung 10 beschränkt und weitere Ausgestaltungen der medizinischen Bildgebungsvorrichtung 36 sind jederzeit denkbar.

Die Magnetresonanzvorrichtung 10 umfasst eine als Magneteinheit 11 ausgebildete Scannereinheit mit einem Grundmagneten 12, einer Gradientenspuleneinheit 13 und einer Hochfrequenzantenneneinheit 14. Zudem weist die medizinische Bildgebungsvorrichtung 36, insbesondere die Magnetresonanzvorrichtung 10, einen Aufnahmebereich 15 auf zu einer Aufnahme eines Objekts und/oder Patienten 16 für eine Magnetresonanzuntersuchung. Der Aufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Aufnahmebereichs 15 jederzeit denkbar.

Für eine Positionierung des Objekts und/oder Patienten 16, insbesondere eines zu untersuchenden Bereichs des Patienten 16, innerhalb des Aufnahmebereichs 15 weist die medizinische Bildgebungsvorrichtung 36, insbesondere die Magnetresonanzvorrichtung 10, eine Patientenlagerungsvorrichtung 17 auf. Die Patientenlagerungsvorrichtung 17 weist eine Basiseinheit 18 und einen bezüglich der Basiseinheit 18 bewegbaren Liegentisch 19 auf. Der Liegentisch 19 ist für eine Positionierung des Objekts und/oder Patienten 16, insbesondere des zu untersuchenden Bereichs des Patienten 16, bewegbar innerhalb des Aufnahmebereichs 15 ausgebildet. Insbesondere ist hierbei der Liegentisch 19 in Richtung einer Längserstreckung des Aufnahmebereichs 15 und/oder in z-Richtung bewegbar gelagert.

Der Grundmagnet 12 der Magneteinheit 11 ist zu einem Erzeugen eines starken und insbesondere konstanten Grundmagnetfelds 20 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 21 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 20 einstellt, ausgebildet. Die Hochfrequenzantenneneinheit 14 wird von einer Hochfrequenzantennensteuereinheit 22 der Magnetresonanzvorrichtung 10 gesteuert und strahlt hochfrequente Magnetresonanzsequenzen in den Aufnahmebereich 15 der Magnetresonanzvorrichtung 10 ein.

Zu einer Steuerung des Grundmagneten 12, der Gradientensteuereinheit 21 und zur Steuerung der Hochfrequenzantennensteuereinheit 22 weist die Magnetresonanzvorrichtung 10 eine Systemsteuereinheit 23 auf. Die Systemsteuereinheit 23 steuert zentral die Magnetresonanzvorrichtung 10, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die medizinische Bildgebungsvorrichtung 36, insbesondere die Magnetresonanzvorrichtung 10, eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Darstellungseinheit 25, beispielsweise auf zumindest einem Monitor, der Benutzerschnittstelle 24 für ein medizinisches Bedienpersonal angezeigt werden. Weiterhin weist die Benutzerschnittstelle 24 eine Eingabeeinheit 26 auf, mittels der Informationen und/oder Parameter während eines Messvorgangs von einem medizinischen Bedienpersonal eingegeben werden können.

Zu einer Positionierung des Patienten 16 weist die medizinische Bildgebungsvorrichtung 36, insbesondere die Magnetresonanzvorrichtung 10, eine Erfassungsvorrichtung 27 und eine Projektionsvorrichtung 28 auf. Die Erfassungsvorrichtung 27 ist zu einem Erfassen einer Position eines zu untersuchenden Bereichs des Objekts und/oder des Patienten 16 für eine Magnetresonanzuntersuchung ausgebildet. Hierzu weist die Erfassungsvorrichtung 27 bevorzugt eine Kamera, insbesondere eine 3D-Kamera 29, auf. Alternativ hierzu kann die Erfassungsvorrichtung 27 auch zwei oder mehr 2D-Kameras umfassen. Die Erfassungsvorrichtung 27 ist im vorliegenden Ausführungsbeispiel fest an einer Decke 30 eines Untersuchungsraums 31, in dem die Magneteinheit 11 der Magnetresonanzvorrichtung 10 angeordnet ist, angeordnet. Dabei ist die Erfassungsvorrichtung 27 in einem Frontbereich 32 vor einer Frontseite 33 der Magneteinheit 11 angeordnet, um den Patienten 16 und/oder den Liegentisch 19 vor einem Einfahren in den Aufnahmebereich 15 zu erfassen. In einer alternativen Ausbildung der Erfindung kann die Erfassungsvorrichtung 27 auch an der Frontseite 33 der Magneteinheit 11 angeordnet sein.

Zur Erfassung des zu untersuchenden Bereichs des Objekts, insbesondere des Patienten 16, wird vom medizinischen Bedienpersonal bevorzugt ein Markierelement 34 an dem zu untersuchenden Bereich des Objekts, insbesondere des Patienten 16, positioniert, das von der Erfassungsvorrichtung 27 erkannt und erfasst wird. Das Markierelement 34 kann beispielsweise einen Finger des medizinischen Bedienpersonals oder auch einen handgeführten Gegenstand, beispielsweise einen Markierstab, umfassen.

Die Projektionsvorrichtung 28 umfasst bevorzugt einen Videoprojektor und/oder eine Laser-Markierungseinheit, die zu einer Projektion und/oder Anzeige einer Feedback-Markierung an der von der Erfassungsvorrichtung 27 erfassten Position des Markierelements 34 ausgebildet ist. Die Feedback-Markierung ist für den Benutzer, insbesondere das medizinische Bedienpersonal, vorgesehen, um eine von der Erfassungsvorrichtung 27 erfasste Position des zu untersuchenden Bereichs zu kontrollieren. Die Projektionsvorrichtung 28 ist im vorliegenden Ausführungsbeispiel fest an einer Decke 30 des Untersuchungsraums 31 angeordnet. Dabei ist die Projektionsvorrichtung 28 in dem Frontbereich 32 vor der Frontseite 33 der Magneteinheit 11 angeordnet, um auf den Patienten 16 vor einem Einfahren in den Aufnahmebereich 15 eine Feedback-Markierung zu projizieren. In einer alternativen Ausbildung der Erfindung kann die Projektionsvorrichtung 28 auch an der Frontseite 33 der Magneteinheit 11 angeordnet sein.

Die medizinische Bildgebungsvorrichtung 36, insbesondere die Magnetresonanzvorrichtung 11, weist des Weiteren eine 3D-Kamera 29 auf, die zu einer Erfassung von 3D-Daten des auf dem Liegentisch 19 positionierten Objekts, insbesondere Patienten 16, ausgebildet ist. Im vorliegenden Ausführungsbeispiel ist die 3D-Kamera 29 von der Erfassungsvorrichtung 27 umfasst.

Zu einer Steuerung der Erfassungsvorrichtung 27 und der Projektionsvorrichtung 28 weist die medizinische Bildgebungsvorrichtung 36, insbesondere die Magnetresonanzvorrichtung 10, eine Recheneinheit 35 auf. Die Recheneinheit 35 ist im vorliegenden Ausführungsbeispiel in die Systemsteuereinheit 23 integriert. In einer alternativen Ausgestaltung kann die Recheneinheit 35 auch separat zur Systemsteuereinheit 23 ausgebildet sein.

Die dargestellte medizinische Bildgebungsvorrichtung 36, insbesondere die Magnetresonanzvorrichtung 10, kann selbstverständlich weitere Komponenten umfassen, die medizinische Bildgebungsvorrichtungen 36, insbesondere Magnetresonanzvorrichtungen 10, gewöhnlich aufweisen. Eine allgemeine Funktionsweise einer medizinischen Bildgebungsvorrichtung 36, insbesondere einer Magnetresonanzvorrichtung 10, ist zudem dem Fachmann bekannt, so dass auf eine detaillierte Beschreibung der weiteren Komponenten verzichtet wird.

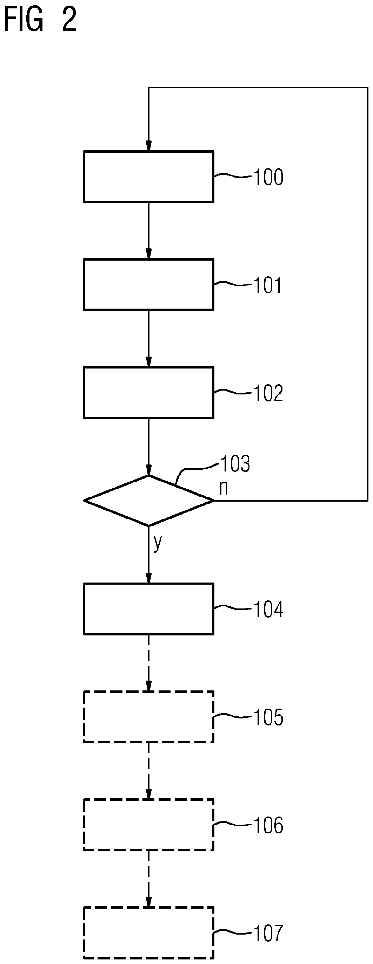

In Fig. 2 ist ein Verfahren zu einem Erfassen einer Position eines zu untersuchenden Bereichs eines Objekts, insbesondere eines Patienten 16, für eine medizinische Bildgebungsuntersuchung dargestellt. Das Verfahren wird von der Recheneinheit 35 der medizinischen Bildgebungsvorrichtung 36, insbesondere der Magnetresonanzvorrichtung 10, ausgeführt, wobei mittels der Recheneinheit 35 die einzelnen Verfahrensschritte des Verfahrens zu einem Erfassen einer Position eines zu untersuchenden Bereichs eines Objekts, insbesondere eines Patienten 16, für eine medizinische Bildgebungsuntersuchung zu steuern. Die Recheneinheit 35 weist hierzu eine entsprechende Steuersoftware und/oder Computerprogramme auf, die in einem Speicher der Recheneinheit 35 gespeichert sind. Bei einem Ausführen der Steuersoftware und/oder der Computerprogramme durch einen Prozessor der Recheneinheit 36 werden die einzelnen Verfahrensschritte entsprechend angesteuert.

Das erfindungsgemäße Verfahren wird während einer Vorbereitung des Objekts, insbesondere des Patienten 16, für eine medizinische Bildgebungsuntersuchung ausgeführt. Hierbei wird am Ende der Vorbereitung, bevor das zu untersuchende Objekt, insbesondere der zu untersuchende Patient 16, in den Aufnahmebereich 15 mittels des Liegentischs 19 eingefahren wird, in einem ersten Verfahrensschritt 100 von dem medizinischen Bedienpersonal der zu untersuchende Bereich des Objekts, insbesondere des Patienten 16, markiert. Das Markieren erfolgt durch ein manuelles Positionieren des Markierelements 34 an dem zu untersuchenden Bereich des Objekts, insbesondere des Patienten 16. Das Markierelement 34 kann dabei einen Finger des medizinischen Bedienpersonals oder auch ein handgeführtes Markierelement, wie beispielsweise einen Markierstab, umfassen.

In einem zweiten Verfahrensschritt 101 erfolgt ein Erfassen der Position des Markierelements 34 mittels der Erfassungsvorrichtung 27. Dabei werden die erfasste Positionsdaten der Erfassungsvorrichtung 27 an die Recheneinheit 35 geleitet und dort ausgewertet und entsprechende Positionsinformationen des Markierelements 34 ermittelt.

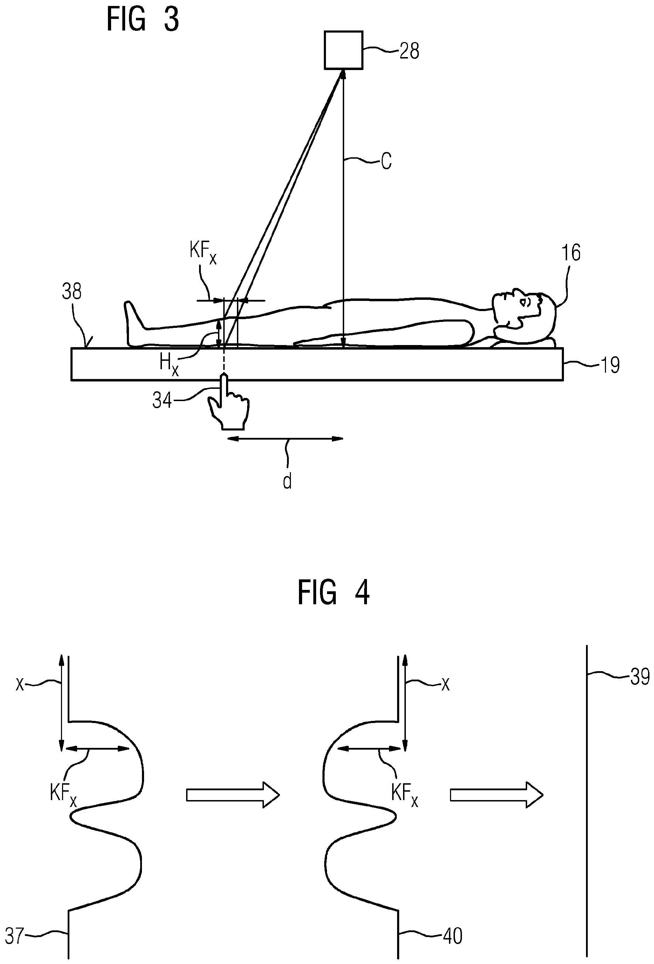

In einem dritten Verfahrensschritt 102 erfolgt ein Projizieren einer Feedback-Markierung an der erfassten Position des Markierelements 34 mittels der Projektionsvorrichtung 28. Die Feedback-Markierung umfasst dabei eine projizierte Markierungslinie 39 in x-Richtung, wobei die Feedback-Markierung, insbesondere die Markierungslinie 39, an der erfassten Position des Markierelements auf das Objekt, insbesondere auf dem Patienten 16, von der Projektionsvorrichtung 16 projiziert wird. Hierzu erhält die Projektionsvorrichtung 28 die Positionsinformationen des Markierelements von der Recheneinheit 35. Die Positionsinformationen des Markierungselements und damit auch die Positionsinformationen der Feedback-Markierung beziehen sich auf eine Liegefläche 38 des Liegentischs 19. Da das Objekt, insbesondere ein Patient 16, eine dreidimensionale Ausdehnung aufweist, liegt bei der Projektion der Feedback-Markierung eine Verzerrung in z-Richtung vor, die abhängig ist von einer Höhe des Profils des Patienten 16 an der Position des Markierelements 34 in z-Richtung (Fig. 3). Um Diese Verzerrung bei der Projektion der Feedback-Markierung zu eliminieren, wird von der Projektionsvorrichtung 28 bei der Generierung und Projektion der Feedback-Markierung, insbesondere der Markierungslinie 39, ein Korrekturfaktor $KF_x$ in z-Richtung berücksichtigt.

Zur Bestimmung und/oder Ermittlung des Korrekturfaktors $KF_x$ werden 3D-Daten des Objekts, insbesondere des Patienten 16, erfasst. Das Erfassen der 3D-Daten erfolgt dabei mittels der 3D-Kamera 29 der Erfassungsvorrichtung 27. Aus den 3D-Daten wird von der Recheneinheit 35 ein 3D-Profil des Objekts, insbesondere des Patienten 16, erstellt und/oder bestimmt. Dabei wird ein 3D-Profil des auf dem Liegentisch 19 liegenden Objekts, insbesondere Patienten 16, erstellt und/oder bestimmt. Von der Recheneinheit 35 wird zudem aus dem 3D-Profil eine Höhe $H_x$ des Objekts, insbesondere des Patienten 16, an der erfassten Position des Markierelements 34 ermittelt und/oder bestimmt. Dabei wird von der Recheneinheit 35 für jede Position in x-Richtung an der Position der Markierungslinie 39 eine Höhe $H_x$ des Objekts, insbesondere des Patienten 16, aus dem 3D-Profil des Objekts, insbesondere des Patienten 16, ermittelt.

Die ermittelte Höhe $H_x$ des Objekts, insbesondere des Patienten 16, an der erfassten Position des Markierelements 34 geht dabei in den Korrekturfaktor $KF_x$ ein. Neben der der ermittelten Höhe $H_x$ des Objekts, insbesondere des Patienten 16, gehen in den Korrekturfaktor $KF_x$ zusätzlich ein Abstand d des Markierelements 34 zu einer Position der Projektionsvorrichtung 28 in z-Richtung und eine Höhe C der Projektionsvorrichtung 28 über dem Liegentisch 19 ein. Der Korrekturfaktor $KF_x$ wird von der Recheneinheit 35 ermittelt. Der Korrekturfaktor $KF_x$ setzt sich dabei wie folgt zusammen:

$$KF_x = \frac{d}{C} H_x$$

Hierbei ist $KF_x$ der Korrekturfaktor und/oder ein Korrekturwert an der Position x entlang der Markierungslinie 37, wobei der Korrekturfaktor $KF_x$ ein additiver Kor-

rekturfaktor und/oder Korrekturwert, der von einer x-Geraden an der x-Position des Markierelements 34 abgezogen wird. $H_x$ ist die Höhe des Objekts, insbesondere des Patienten 16, in x-Richtung bezogen auf die Liegefläche 38 des Liegentischs 19 (Fig. 3 und 4).

Durch den Korrekturfaktor $KF_x$ wird für jede x-Position der Markierungslinie ohne Korrektur 37 ein Korrekturwert berechnet und bei der Projektion der Feedback-Markierung, insbesondere bei der Projektion einer korrigierten Markierungslinie 39 der Feedback-Markierung, berücksichtigt. In Fig. 4 ist zunächst eine Projektion der Feedback-Markierung, insbesondere der Markierungslinie 37, ohne Korrekturfaktor $KF_x$ auf einem Beinbereich des Patienten 16 dargestellt, wobei die Feedback-Markierung, insbesondere die Markierungslinie 37, eine Abhängigkeit von der Höhe $H_x$ des Patienten 16 entlang der x-Richtung und/oder der Markungslinie 37 zeigt. Die Linie 40 zeigt eine von der Projektionsvorrichtung 28 generierte Feedback-Markierung vor einer Projektion auf das Objekt, im vorliegenden Ausführungsbeispiel auf die Beine des Patienten 16, bei der der Korrekturfaktor $KF_x$ berücksichtigt ist. Diese von der Projektionsvorrichtung 28 generierte Linie 40 wird von der Projektionsvorrichtung 28 als korrigierte Markierungslinie 39 auf das Objekts, im vorliegenden Ausführungsbeispiel auf die Beine des Patienten 16, projiziert. Durch den Korrekturfaktor $KF_x$ wird die Markierungslinie 37 derart korrigiert, dass sie unabhängig von einer Höhe $H_x$ des Patienten 16 als gerade Linie, insbesondere als gerade und/oder korrigierte Markierungslinie 39, auf dem Patienten 16 projiziert wird.

Zusätzlich wird aus den 3D-Daten eines Patienten 16 ein Augenbereich des Patienten 16 ermittelt. Dieser Augenbereich des Patienten 16 wird bei der anschließenden Projektion der Feedback-Markierung, insbesondere der Markierungslinie 37, von einem Projektionsbereich der Projektionsvorrichtung 28 ausgeschlossen.

In einem weiteren, vierten Verfahrensschritt 103 wird überprüft, ob die projizierte Feedback-Markierung, insbesondere die korrigierte Markierungslinie 39, mit der Position des Markierelements 34 und damit mit der Position des zu untersuchenden Bereichs des Objekts, insbesondere des Patienten 16, übereinstimmt. Beispielsweise kann nach Erhalt der Feedback-Markierung, insbesondere der korrigierten Markierungslinie 39, sofern diese nicht mit einer Position des Markierelements 34 und damit des zu untersuchenden Bereichs des Objekts, insbesondere des Patienten 16, übereinstimmt, durch eine manuelle Neupositionierung des Markierelements 34 der Erfassungsvorrichtung 27 mitgeteilt werden, dass die erfasste Position nicht korrekt war. Ergibt die Überprüfung, dass die projizierte Feedback-Markierung nicht mit der Position des Markierelements 34 übereinstimmt, werden der Verfahrensschritt 101 des Erfassens der Position des Markierelements 34 und der Verfahrensschritt 102 des Projizierens der Feedback-Markierung an der erfassten Position des Markierelements 34 wiederholt ausgeführt. Dabei werden der Verfahrensschritt 101 des Erfassens der Position des Markierelements 34 und der Verfahrensschritt 102 des Projizierens der Feedback-Markierung an der erfassten Position des Markierelements 34 so lange wiederholt, bis die Feedback-Markierung mit der Position des Markierelements 34 übereinstimmt.

Stimmt dagegen in diesem Verfahrensschritt 103 die projizierte Feedback-Markierung mit der Position des Markierelements 34 und damit mit der Position des zu untersuchenden Bereichs des Objekts, insbesondere des Patienten 16, überein, kann dies der Erfassungsvorrichtung 27 beispielsweise durch ein Beibehalten der Position des Markierelements 34 und/oder ein komplettes Wegnehmen des Markierelements 34 aus dem Erfassungsbereich der Erfassungsvorrichtung 27 signalisiert werden. Sobald die Position der Feedback-Markierung mit der Position des Markierelements 34 und damit des zu untersuchenden Bereichs des Patienten 16 für den Benutzer übereinstimmt und der Benutzer der Erfassungsvorrichtung 28 signalisiert, dass die Position korrekt ist, wird in einem weiteren, fünften Verfahrensschritt 104 die Position des Markierelements 34 als die Position des zu untersuchenden Bereichs des Objekts, insbesondere des Patienten 16, festgelegt.

In einem weiteren optionalen Verfahrensschritt 105 wird anhand der erfassten Position des zu untersuchenden Bereichs des Objekts, insbesondere des Patienten 16, eine Untersuchungsposition des Liegentischs 19 für die anstehende medizinische Bildgebungsuntersuchung, insbesondere Magnetresonanzuntersuchung, berechnet. In einem weiteren optionalen Verfahrensschritt 106 wird anschließend der Liegentisch 19 zusammen mit dem Objekt, insbesondere mit dem Patienten 16, in diese Untersuchungsposition gefahren für eine Erfassung von medizinischen Bildgebungsdaten, insbesondere von Magnetresonanzdaten, des zu untersuchenden Bereichs. In dieser Untersuchungsposition befindet sich der zu untersuchende Bereich des Objekts, insbesondere des Patienten 16, innerhalb des Aufnahmebereichs 15, insbesondere des Isozentrums, der medizinischen Bildgebungsvorrichtung 36, insbesondere der Magnetresonanzvorrichtung 10. Anschließend wird in einem weiteren optionalen Verfahrensschritt 107 die medizinische Bildgebungsuntersuchung, insbesondere die Magnetresonanzuntersuchung, des zu untersuchenden Bereichs des Objekts, insbesondere des Patienten 16, durchgeführt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren zu einem Erfassen einer Position eines zu untersuchenden Bereichs eines Objekts für eine medizinische Bildgebungsuntersuchung, umfassend die folgenden Verfahrensschritte:

   - Positionieren eines Markierelements an dem zu untersuchenden Bereich des Objekts, wobei das Positionieren des Markierelements manuell durch ein medizinisches Bedienpersonal erfolgt,
   - Erfassen der Position des Markierelements mittels einer Erfassungsvorrichtung,
   - Projizieren einer Feedback-Markierung an der erfassten Position des Markierelements mittels einer Projektionsvorrichtung, wobei bei der Ermittlung einer Projektionsposition der Feedback-Markierung ein Korrekturfaktor in z-Richtung berücksichtigt wird,
   - Wiederholen des Erfassens der Position des Markierelements und des Projizierens der Feedback-Markierung, bis die Feedback-Markierung mit der Position des Markierelements übereinstimmt, und
   - Festlegen der Position des Markierelements als Position des zu untersuchenden Bereiches des Objekts.

2. Verfahren nach Anspruch 1, **dadurch gekennzeichnet, dass** zur Ermittlung des Korrekturfaktors 3D-Daten des Objekts erfasst werden und anhand der 3D-Daten ein 3D-Profil des Objekts bestimmt wird.

3. Verfahren nach Anspruch 2, **dadurch gekennzeichnet, dass** aus dem 3D-Profil eine Höhe des Objekts an der erfassten Position des Markierelements ermittelt wird.

4. Verfahren nach einem der Ansprüche 2 oder 3, **dadurch gekennzeichnet, dass** die Feedback-Markierung zur Festlegung der Position des zu untersuchenden Bereichs in z-Richtung eine projizierte Markierungslinie in x-Richtung auf dem Objekt umfasst, wobei für jede Position in x-Richtung der Markierungslinie eine Höhe des Objekts aus dem 3D-Profil des Objekts ermittelt wird.

5. Verfahren nach einem der Ansprüche 2 bis 4, **dadurch gekennzeichnet, dass** der Korrekturfaktor in z-Richtung eine Höhe des Objekts an der erfassten Position des Markierelements umfasst.

6. Verfahren nach einem der vorhergehenden Ansprüche, **dadurch gekennzeichnet, dass** der Korrekturfaktor einen Abstand des Markierelements zu einer Position der Projektionsvorrichtung in z-Richtung und eine Höhe der Projektionsvorrichtung über dem Liegentisch umfasst.

7. Verfahren nach einem der vorhergehenden Ansprüche, **dadurch gekennzeichnet, dass** anhand von 3D-Daten eines Patienten ein Augenbereich des Patienten ermittelt wird, wobei der Augenbereich bei der Projektion der Feedback-Markierung von einem Projektionsbereich der Projektionsvorrichtung ausgeschlossen wird.

8. Verfahren nach einem der vorhergehenden Ansprüche, **dadurch gekennzeichnet, dass** anhand der erfassten Position des zu untersuchenden Bereichs des Objekts eine Untersuchungsposition des Liegentischs berechnet wird.

9. Verfahren nach Anspruch 8, **dadurch gekennzeichnet, dass** der Liegentisch automatisch in die Untersuchungsposition gefahren wird für eine Erfassung von medizinischen Bildgebungsdaten des zu untersuchenden Bereichs des Objekts.

10. Medizinische Bildgebungsvorrichtung umfassend eine Erfassungsvorrichtung, die zu einer Erfassung einer Position eines Markierelements ausgebildet ist, eine Projektionsvorrichtung, die zu einer Projektion einer Feedback-Markierung an der erfasste Position des Markierelement ausgebildet ist, eine Kamera und eine Recheneinheit, wobei die medizinische Bildgebungsvorrichtung zu einer Ausführungen eines Verfahrens zu einem Erfassen einer Position eines zu untersuchenden Bereichs eines Objekts für eine medizinische Bildgebungsuntersuchung nach einem der Ansprüche 1 bis 9 ausgebildet ist.

11. Medizinische Bildgebungsvorrichtung nach Anspruch 10, **gekennzeichnet durch** eine Scannereinheit mit einer Frontseite, wobei die Erfassungsvorrichtung und/oder die Projektionsvorrichtung in einem Frontbereich vor der Frontseite der Scannereinheit angeordnet ist oder sind.

12. Medizinische Bildgebungsvorrichtung nach Anspruch 11, **dadurch gekennzeichnet, dass** die Erfassungsvorrichtung und/oder die Projektionsvorrichtung fest an einer Decke eines Untersuchungsraums und/oder an der Frontseite der Scannereinheit angeordnet ist oder sind.

13. Medizinische Bildgebungsvorrichtung nach einem der Ansprüche 10 bis 12, **dadurch gekennzeichnet, dass** die Erfassungsvorrichtung die Kamera umfasst.

14. Medizinische Bildgebungsvorrichtung nach einem der Ansprüche 10 bis 13,
**gekennzeichnet durch** eine Magnetresonanzvorrichtung.

15. Computerprogrammprodukt, welches ein Programm umfasst und direkt in einen Speicher einer programmierbaren Recheneinheit ladbar ist, mit Programmmitteln, um ein Verfahren zu einem Erfassen einer Position eines zu untersuchenden Bereichs eines Objekts für eine medizinische Bildgebungsuntersuchung nach einem der Ansprüche 1 bis 9 auszuführen, wenn das Programm in der Recheneinheit ausgeführt wird.

FIG 1

EP 4 509 037 A1

FIG 2

# FIG 3

# FIG 4

**EP 4 509 037 A1**

Europäisches Patentamt
European Patent Office
Office européen des brevets

# EUROPÄISCHER RECHERCHENBERICHT

**Nummer der Anmeldung**

**EP 23 19 2000**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2014 210938 A1 (SIEMENS AG [DE]) 17. Dezember 2015 (2015-12-17) | 1-15 | INV. A61B5/00 |
| Y | * Absätze [0002], [0029], [0033], [0039], [0043] – [0045], [0051], [0055]; Abbildungen 1-7 * | 1-15 | A61B5/055 A61B6/04 |
| | ----- | | |
| Y,D | DE 10 2021 202978 A1 (SIEMENS HEALTHCARE GMBH [DE]) 29. September 2022 (2022-09-29) * Ansprüche 1,21,22; Abbildungen 1,3 * | 1-15 | |
| | ----- | | |
| Y | DE 10 2014 216718 A1 (SIEMENS AG [DE]) 25. Februar 2016 (2016-02-25) * Absätze [0054] – [0066]; Abbildungen 1,2 * | 1-15 | |
| | ----- | | |
| Y | DE 10 2014 205702 A1 (SIEMENS AG [DE]) 1. Oktober 2015 (2015-10-01) * Absätze [0036], [0050]; Abbildung 1 * | 7,15 | |
| | ----- | | |

| RECHERCHIERTE SACHGEBIETE (IPC) |
|---|
| A61B |

Der vorliegende Recherchenbericht wurde für alle Patentansprüche erstellt

| Recherchenort | Abschlußdatum der Recherche | Prüfer |
|---|---|---|
| Den Haag | 12. Januar 2024 | Lommel, André |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

17

**ANHANG ZUM EUROPÄISCHEN RECHERCHENBERICHT
ÜBER DIE EUROPÄISCHE PATENTANMELDUNG NR.**

EP 23 19 2000

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-01-2024

| Im Recherchenbericht angeführtes Patentdokument | Datum der Veröffentlichung | Mitglied(er) der Patentfamilie | | Datum der Veröffentlichung |
|---|---|---|---|---|
| DE 102014210938 A1 | 17-12-2015 | DE | 102014210938 A1 | 17-12-2015 |
| | | US | 2015351709 A1 | 10-12-2015 |
| DE 102021202978 A1 | 29-09-2022 | DE | 102021202978 A1 | 29-09-2022 |
| | | US | 2022304634 A1 | 29-09-2022 |
| DE 102014216718 A1 | 25-02-2016 | CN | 106572829 A | 19-04-2017 |
| | | DE | 102014216718 A1 | 25-02-2016 |
| | | EP | 3182895 A1 | 28-06-2017 |
| | | US | 2017224298 A1 | 10-08-2017 |
| | | WO | 2016026758 A1 | 25-02-2016 |
| DE 102014205702 A1 | 01-10-2015 | CN | 104939924 A | 30-09-2015 |
| | | DE | 102014205702 A1 | 01-10-2015 |
| | | KR | 20150112830 A | 07-10-2015 |
| | | US | 2015272505 A1 | 01-10-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102021202978 A1